# FASCICULE DE BREVET EUROPEEN

(11) **EP 2 201 052 B1**
(45) Date de publication et mention de la délivrance du brevet: **12.12.2012**
(21) Numéro de dépôt: 08845161.2
(22) Date de dépôt: 08.10.2008
(51) Int. Cl.: C08F 246/00, G01N 33/68, B01J 20/26, C08F 220/28, C08F 226/06, C08F 222/10

(54) **POLYMERES A EMPREINTES MOLECULAIRES POUR LA RECONNAISSANCE DU GLUTATHION GSH, LEURS PROCEDES DE PREPARATION ET UTILISATIONS**
MOLEKULAR GEPRÄGTE POLYMERE ZUR ERKENNUNG VON GLUTATHION-GSH, VERFAHREN ZU IHRER HERSTELLUNG UND VERWENDUNGEN DAVON
MOLECULARLY IMPRINTED POLYMERS FOR THE RECOGNITION OF GLUTATHIONE GSH, METHODS FOR PREPARING SAME AND USES THEREOF

(30) Priorité: 10.10.2007 FR 0758193
(43) Date de publication de la demande: 30.06.2010
(73) Titulaire: Polyintell, 27100 Val de Reuil (FR)
(72) Inventeur: PEROLLIER, Céline, F-76000 Rouen (FR); BAYOUDH, Sami, F-76000 Rouen (FR)
(74) Mandataire: Le Coupanec, Pascale A.M.P.
(86) Numéro de dépôt international: PCT/FR2008/051815
(87) Numéro de publication internationale: WO 2009/056716

(56) Documents cités:
- SHARMA P S ET AL: "Highly Sensitive and Selective Detection of Creatinine by Combined Use of MISPE and a Complementary MIP-Sensor" CHROMATOGRAPHIA ; AN INTERNATIONAL JOURNAL FOR RAPID COMMUNICATION IN CHROMATOGRAPHY, ELECTROPHORESIS AND ASSOCIATED TECHNIQUES, VIEWEG VERLAG, WI, vol. 65, no. 7-8, 6 février 2007 (2007-02-06), pages 419-427, XP019496249 ISSN: 1612-1112
- CHIANELLA I ET AL: "MIP-based solid phase extraction cartridges combined with MIP-based sensors for the detection of microcystin-LR" BIOSENSORS & BIOELECTRONICS, ELSEVIER SCIENCE PUBLISHERS, BARKING, GB, vol. 18, no. 2-3, mars 2003 (2003-03), pages 119-127, XP009090174 ISSN: 0956-5663

## Description

La présente invention se rapporte au domaine des polymères à empreintes moléculaires (encore appelés « molecularly inprinted polymers » ou « MIPs » en langue anglaise) utiles pour la reconnaissance de molécules cibles.

Elle concerne plus particulièrement de nouveaux MIPs adaptés à la reconnaissance moléculaire sélective du glutathion GSH et/ou de l'un de ses analogues, et en particulier utiles pour le traitement de milieux comprenant notamment un mélange de glutathion GSH et/ou de l'un de ses analogues avec des adduits du GSH.

Le glutathion existe naturellement sous deux formes chimiques, à savoir la forme oxydée (GSSG) et la forme réduite (GSH) qui représente environ 98 à 99 % du glutathion total.

Le glutathion réduit (ou γ-L-glutamyl-L-cystéinylglycine, noté glutathion GSH ou simplement GSH) est un tripeptide synthétisé par toutes les cellules de l'organisme et constitue le composé comportant un thiol (-SH) libre de bas poids moléculaire le plus abondant dans les cellules eucaryotes. Il participe de façon prépondérante aux mécanismes de défense cellulaire grâce à sa fonction sulfhydryle qui est à l'origine de sa grande réactivité et de son puissant pouvoir réducteur. La concentration intracellulaire en GSH est ainsi considérée par certains auteurs comme un véritable marqueur de l'état de santé d'un individu.

En conséquence, le GSH est utilisé de façon routinière pour la mise en oeuvre de tests de toxicologie *in vitro,* comme agent chimique de piégeage utile pour la caractérisation et l'étude mécanistique des métabolites réactifs de molécules actives.

A titre illustratif et non limitatif de molécules actives pouvant être soumises à de tels tests de toxicologie au glutathion, on peut par exemple citer les additifs alimentaires, les produits phytosanitaires, les actifs cosmétiques, les candidats-médicaments, les médicaments humains et vétérinaires, et leurs métabolites actifs.

Par exemple, lorsqu'une molécule est identifiée comme potentiellement intéressante en thérapie ou pour le diagnostic, elle est soumise à des tests de toxicité en « *early stage* », afin de mettre en évidence au plus tôt sa toxicité éventuelle et de permettre aux laboratoires de prendre le plus rapidement possible une décision sur son développement.

La prédiction préclinique des candidats médicaments est toutefois difficile, et ce d'autant plus lorsque ceux-ci sont associés à des réactions idiosyncratiques. La plupart des candidats médicaments de ce type forme des métabolites réactifs, qui peuvent réagir avec des nucléophiles endogènes, à l'image du GSH.

L'analyse des adduits covalents formés entre ces métabolites et le GSH donne accès à des informations sur la nature et la formation de ces métabolites réactifs. C'est la raison pour laquelle le test de toxicologie au glutathion impliquant ces métabolites réactifs a été adopté par un grand nombre de sociétés pharmaceutiques.

Plus précisément, les tests de toxicologie sur des candidats médicaments faisant intervenir le GSH consistent à mettre en présence, d'une part, le candidat médicament et des microsomes (permettant la formation des principaux métabolites du candidat-médicament) et, d'autre part, une quantité très importante (généralement de 20 à 500 fois plus importante) de GSH, afin de générer les différents adduits du GSH avec le candidat-médicament et/ou certains de ses métabolites. La quantification et la détermination de la nature et de la structure des adduits formés à partir du candidat médicament et/ou certains de ses métabolites avec le GSH fournissent des informations précieuses aux chercheurs en charge de la recherche de nouveaux médicaments, et permettent notamment de sélectionner les meilleurs candidats médicaments.

Toutefois, la présence de quantités importantes de glutathion (sous forme GSH et GSSG) dans le milieu d'analyse associé rend très difficile, voire impossible, la séparation, la quantification et l'étude des adduits formés lors de ces tests de toxicologie et on se heurte, par conséquent, à des problèmes analytiques pour exploiter les résultats.

Plusieurs méthodes ont déjà été élaborées pour tenter d'améliorer la détection des adduits du GSH formés.

Une première technique consiste à utiliser un candidat médicament radiomarqué. Selon cette première méthode, les métabolites du candidat médicament seront eux-aussi radiomarqués, et par conséquent leurs adduits avec le GSH le seront également. L'utilisation d'un système chromatographique couplé avec un détecteur radiosensitif permet alors de séparer le candidat médicament, ses métabolites et ses adduits avec le GSH des molécules de glutathion présentes en excès, ces dernières n'étant pas détectées puisqu'elles ne sont pas radiomarquées. Cette technique implique donc le marquage préalable des candidats médicaments, ce qui n'est pas toujours réalisable, et a pour inconvénient d'être relativement longue à mettre en oeuvre et onéreuse.

Une deuxième méthode, qui est celle la plus largement employée de nos jours, consiste à utiliser du glutathion marqué (à savoir radiomarqué, fluorescent ou modifié chimiquement). Selon cette deuxième méthode, lorsque le GSH réagira avec le candidat médicament et/ou certains de ses métabolites, il formera un adduit également marqué. Cette voie implique toutefois de trouver une méthode analytique permettant la séparation du glutathion marqué (généralement de 20 à 500 fois plus présent) des adduits du GSH marqués.

A titre de glutathion radiomarqué, il est connu d'utiliser du GSH tritié, permettant de quantifier directement les pics correspondant aux adduits. Cependant, la séparation entre le GSH et ses adduits n'est pas aisée et les résultats sont insatisfaisants du point de vue de la sensibilité (Thompson D.C. Chem. Res. Toxicol. 1995, 8, 55-60). De plus, l'emploi de réactifs radiomarqués implique des aménagements spéciaux et une gestion spécifique des déchets, ainsi que l'achat de réactifs onéreux.

Pour obtenir du glutathion fluorescent, le document US 2005/0186651 divulgue pour sa part un procédé consistant à dérivatiser la fonction amine du GSH avec un groupement dansyl (noté dGSH). Il a cependant été observé que l'adduit des métabolites de l'acétominophénol coélue avec le dGSH et qu'il existe une fluorescence résiduelle du dGSH à cause de sa forte proportion. La présence de dGSSGd gênant pour l'analyse a également été détectée, et c'est pourquoi du dithiothréitol (pouvant être gênant dans le cas de l'étude de formation de disulfure avec les molécules-candidats) doit être en outre ajouté pour éviter la formation de ce dimère.

Enfin, il est également connu d'utiliser du GSH modifié par un ammonium quaternaire (QA-GSH) de façon à quantifier de façon semi-quantitative les adduits du GSH (Soglia J.R. Chem. Res. Toxicol. 2006, 19, 480-490). Le QA-GSH peut ainsi être additionné aux échantillons issus des tests de toxicité avant l'analyse, à titre d'étalon interne.

D'une manière générale, cette seconde alternative qui requiert la synthèse ou l'achat de GSH marqué, ne donne pas non plus totalement satisfaction pour analyser les adduits du GSH.

Il demeure donc un besoin d'une méthode simple, sensible, rapide, peu coûteuse, adaptable aux tests de criblage à haut débit et fiable permettant d'exploiter les résultats des tests de toxicologie au glutathion précités, tout en s'affranchissant du marquage de la molécule active ou du GSH.

Il demeure en particulier un besoin de faciliter la séparation, la détection et l'analyse des adduits du GSH formés lors de ces tests, afin d'évaluer le plus rapidement possible la toxicité d'une molécule active.

La présente invention vise précisément à satisfaire ces besoins.

De manière inattendue, les inventeurs ont ainsi constaté qu'il était possible d'élaborer des polymères à empreintes moléculaires (MIPs) dédiés à la reconnaissance sélective du glutathion GSH et/ou de ses analogues.

Ainsi, la présente invention concerne, selon un premier de ses aspects, un procédé de préparation d'un polymère à empreintes moléculaires (MIP) dédié à la reconnaissance sélective de la forme réduite du glutathion, GSH, ou analogue, 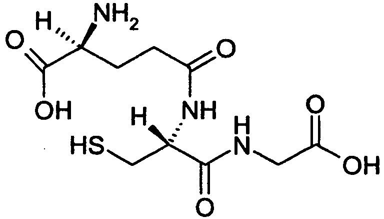 comprenant au moins :
- la copolymérisation de monomère(s) destiné(s) à former la matrice de ladite empreinte moléculaire avec au moins un monomère de formule générale (I) :

   X-(R)ₙ-Y-G (I),

   dans laquelle :
- X représente un groupe monovalent polymérisable,
- n est égal à 0 ou 1,
- R représente une chaîne hydrocarbonée en C₁-C₁₀, notamment en C₁-C₅, linéaire, ramifiée ou cyclique, saturée ou insaturée, éventuellement interrompue par un ou plusieurs hétéroatomes choisis parmi N, O et S, et optionnellement substituée,
- Y représente une fonction de liaison labile impliquant la fonction sulfhydryle du glutathion GSH ou de l'un de ses analogues,
- G représente le résidu du glutathion GSH ou de l'un de ses analogues correspondants,
dans des conditions propices à la formation de ladite empreinte moléculaire, et
- la libération du résidu G par clivage de la fonction de liaison Y.

L'invention concerne également les polymères à empreintes moléculaires (MIPs) susceptibles d'être obtenus par le procédé décrit précédemment.

La présente demande divulgue un polymère à empreintes moléculaires (MIP) comprenant au moins deux sites de reconnaissance pour une entité glutathion GSH et/au pour l'un de ses analogues, dont au moins un site de reconnaissance apte à interagir avec la fonction sulfhydryle de ladite entité glutathion GSH et/ou de l'un de sesdits analogues.

Ledit site de reconnaissance peut être porteur d'un motif -S-S-Z, -S-Z étant un résidu destiné à être déplacé lors de l'interaction du glutathion GSH et/ou de l'un de ses analogues avec ledit MIP, notamment lors de l'interaction de la fonction sulfhydryle -SH du glutathion GSH et/ou de l'un de ses analogues avec ledit site de reconnaissance. Z peut par exemple être un radical aryle, éventuellement substitué contenant de 4 à 10 chaînons, ou un radical hétéroaryle, éventuellement substitué contenant de 4 à 10 chaînons, dont 1 à 4 hétéroatomes choisis parmi O, S, N, NR, avec R représentant H ou alkyle lui-même éventuellement substitué. Z peut notamment représenter

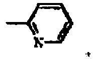

La présente demande divulgue encore un polymère à empreinte moléculaire (MIP) comprenant :
- un ou plusieurs sites de reconnaissance pour une entité glutathion GSH et/ou pour l'un de ses analogues, et
- au moins un résidu G tel que défini précédemment,
ledit résidu G étant piégé dans la cavité dudit polymère à empreinte moléculaires *via* une interaction de sa fonction sulfhydryle native avec l'un desdits sites de reconnaissance.

L'invention concerne également, selon un autre de ses aspects, un procédé de traitement d'un milieu susceptible de comprendre, ou comprenant, au moins du glutathion GSH et/ou l'un de ses analogues, comprenant au moins :
(a) une étape de mise en contact dudit milieu avec au moins un polymère à empreintes moléculaires conforme à l'invention dans des conditions propices à l'extraction du glutathion GSH et/ou de ses analogues, si présents, par ladite empreinte moléculaire,
(b) la séparation dudit polymère à empreintes moléculaires du milieu ainsi traité, et
(c) le cas échéant, la libération du glutathion GSH et/ou de ses analogues dudit polymère à empreintes moléculaires résultant de l'étape (b).

Selon un mode de réalisation, ledit milieu peut être un milieu complexe susceptible de comprendre, outre du glutathion GSH et/ou l'un de ses analogues, au moins un ou plusieurs adduits du GSH.

Ainsi, les MIPs conformes à la présente invention s'avèrent notamment particulièrement avantageux sur le plan analytique lorsqu'il est recherché à détecter la présence, voire à doser la quantité d'adduits du GSH formés en présence d'une quantité importante de GSH et/ou de l'un de ses analogues.

Le MIP selon l'invention, qui est plus particulièrement sélectif vis-à-vis du GSH et/ou de l'un de ses analogues, permet d'extraire de façon plus sélective celui-ci d'un tel milieu et facilite ainsi la caractérisation ultérieure des adduits du GSH susceptibles d'y exister conjointement.

L'invention concerne donc également l'utilisation d'au moins un polymère à empreintes moléculaires (MIP) conforme à l'invention pour le traitement d'un milieu susceptible de comprendre, ou comprenant, au moins du glutathion GSH et/ou l'un de ses analogues et des adduits du GSH.

Outre cette application particulièrement intéressante lors de la mise en oeuvre de tests de toxicologie au glutathion, les MIPs selon l'invention peuvent également constituer un outil simple et de mise en oeuvre aisée pour le dosage du GSH et/ou de l'un de ses analogues.

Ainsi, l'invention concerne également l'utilisation d'au moins un polymère à empreintes moléculaires (MIP) conforme à l'invention à des fins d'extraction, de détection, de séparation, de purification, d'absorption, d'adsorption, de rétention ou de libération contrôlée de la forme libre de glutathion GSH et/ou de l'un de ses analogues.

Les avantages de l'invention sont en outre les avantages liés à l'utilisation de polymères à empreintes moléculaires, à savoir affinité et sélectivité élevées, prix avantageux, simplicité de la préparation et stabilités chimique, mécanique, thermique, qui autorise son utilisation notamment dans des conditions où la chaîne du froid n'est pas respectée, et un stockage longue durée sans perte d'efficacité.

### Définitions :

Dans le cadre de la présente invention, on entend désigner par :
- « adduit », une espèce chimique AB dont chaque entité moléculaire est formée par combinaison directe de deux entités moléculaires distinctes A et B,
- « adduit du GSH », un adduit formé par combinaison directe, d'une part, d'une molécule active (à l'image par exemple d'un additif alimentaire, d'un produit phytosanitaire, d'un actif cosmétique, d'un candidat médicament, ou d'un médicament humain ou vétérinaire) ou de l'un de ses métabolites avec, d'autre part, la fonction sulfhydryle du GSH ou de l'un de ses analogues,
- « analogue », un composé qui a été soumis à une modification chimique, tout en maintenant ses caractéristiques structurelles principales, comme par exemple son squelette. A titre de modifications chimiques, on peut notamment citer par exemple la modification d'un ou plusieurs substituants, notamment à des fins de marquage, ou encore la protection de fonctions réactives.

A titre d'analogue d'un composé, on peut également citer les formes marquées, et en particulier radiomarquées, dudit composé. Le marquage peut notamment être réalisé au moyen d'un chromophore, par exemple fluorescent, à l'image du dansyl par exemple.
- « analogue du GSH », un analogue tel que défini précédemment, dans la mesure où il conserve la fonction sulhydryle native du GSH, de manière qu'il puisse manifester, au même titre que le GSH, l'affinité sélective pour les MIPs selon l'invention,
- « fonction de liaison labile », une liaison covalente pouvant être coupée sous des conditions relativement douce et/ou sélective. Une fonction de liaison labile peut être rompue de façon sélective dans des conditions telles que la rupture des autres liaisons covalentes soit évitée. Par exemple, une liaison disulfure -SS- est susceptible d'être rompue en présence d'un thiol ou encore par irradiation sélective par radiation électromagnétique à une longueur d'ondes spécifique, sans provoquer de rupture des autres liaisons, telles les liaisons carbone-carbone, carbone-oxygène, carbone-soufre, carbone-azote, susceptibles d'être également présentes dans la molécule ou par un agent réducteur (phosphine par exemple).

- « site de reconnaissance », site existant au niveau de la cavité de la matrice du MIP qui intervient effectivement dans la reconnaissance d'une espèce.
- « milieu complexe », un milieu comprenant, outre du glutathion GSH et/ou l'un de ses analogues, au moins une ou plusieurs autres entités annexes comme par exemple un ou plusieurs adduits du GSH.

A titre d'exemple de milieu complexe selon l'invention, on peut notamment citer un milieu d'incubation d'une molécule active mise en oeuvre dans un test de toxicologie au glutathion, comme par exemple un milieu d'incubation d'un candidat-médicament. Dans un milieu complexe de ce type, le glutathion GSH et/ou l'un de ses analogues est présent en des quantités très importantes et est susceptible de gêner l'analyse des adduits du GSH.
- Par « extraction d'une (ou plusieurs) espèce(s) par reconnaissance moléculaire de la (ou des) espèce(s) » s'entend au sens de l'invention une étape au cours de laquelle l'interaction de la (ou des) espèce(s) avec les sites de reconnaissance d'un MIP est suffisante pour conduire à la formation d'un complexe composé du MIP doté, dans tout ou partie de ses sites de reconnaissance, de la (ou des)dite(s) espèce(s).
- Par « libération d'une (ou plusieurs) espèce(s) » s'entend au sens de l'invention une étape au cours de laquelle le complexe formé lors de l'extraction de la (ou des) espèce(s) se dissocie, par exemple suite à une modification des conditions d'oxydation, de réduction, de pH, de salinité, de température, de débit, de pression, ou de polarité des solvants, conduisant à la présence de la (ou des) molécule(s) cible(s) sous une forme libre en solution.

Au sens de l'invention, le terme « affinité » désigne l'aptitude d'une espèce à interagir avec un site de reconnaissance d'un MIP. Une forte affinité traduit ainsi, au sens de l'invention, une forte aptitude pour cette espèce à interagir avec au moins un site de reconnaissance d'un MIP.

Par interaction s'entend, au sens de l'invention, la formation de liaisons faibles (par exemple de type liaisons de Van der Waals, liaisons hydrogène, liaisons pi donneur-pi accepteur, ou interactions hydrophobiques) et/ou de liaisons fortes (par exemple de type liaisons ionique, liaisons covalentes, ou encore liaisons iono-covalentes, et de préférence de type liaison covalente labile).

### POLYMERE A EMPREINTES MOLECULAIRES

L'étape de polymérisation du MIP autour d'une entité patron fait appel à des techniques en soi connues de l'homme de l'art. On peut ainsi se rapporter à l'article Peter A.G. Cormack et al., Journal of Chromatography B, 804 (2004) 173-182, qui Présente une revue des techniques disponibles autour des aspects de la polymérisation de MIPs.

Plus précisément il existe principalement deux approches (dites « classiques » dans la suite) possibles pour faire des MIPs, l'approche covalente développée par Wulff dans le document US 4 127 730 et l'approche non covalente développée par Mosbach dans le document US 5 110 833. Ces deux approches peuvent aussi être combinées.

Il est ainsi possible d'utiliser la première approche de type covalente pour la préparation du MIP et la deuxième approche pour obtenir une reconnaissance par des interactions non covalentes, comme cela est par exemple divulgué dans M.J. Whitcombe et al. « A New Method for the Introduction of Récognition Site Functionality into Polymers prepared by molecular Imprinting : Synthesis and Characterization of Polymeric Receptors for Cholesterol » J. Am. Chem. Soc., 1995, 117, 7105-7111.

Il est également possible d'utiliser les première et deuxième approches pour la préparation du MIP, ainsi que pour obtenir la reconnaissance par des interactions covalentes et non covalentes simultanément pour une même molécule cible. Ainsi, l'interaction se déroule au moins en deux sites distincts du site de reconnaissance comme cela est par exemple divulgué dans Wulff G. et al. Macromol. Chem. Phys. 1989, 190, 1717 et 1727.

Le document Liu Wang et al. « Capacitive Biosensor for Glutathione Detection Based on Electropolymerized Molecularly Imprinted Polymer and Kinetic Investigation of the Recognition Process » ELectroanalysis 2005, 17(11), p.969-977 illustre également une approche dite "classique" pour faire des MIPs, ceux-ci étant obtenus par électropolymérisation d'ortho-phénylènediamines en présence de GSH.

Une troisième approche (dite « semi-covalente » dans la suite) consiste à utiliser pour la synthèse des MIPs des monomères spécifiques selon la (ou les) molécule(s) cible(s) visée(s), et en particulier au moins en partie des monomères dérivés d'une molécule cible, jouant ainsi en partie le rôle du polymère de ta matrice et en partie le rôle de l'entité patron. Autrement dit, une partie de ces monomères, une fois polymérisée, a vocation à être éliminée de sorte à donner naissance aux sites de reconnaissance.

Les polymères à empreintes moléculaires convenant à la mise en oeuvre du procédé selon l'invention sont précisément obtenus suivant cette approche semi-covalente.

Plus précisément, ils peuvent être obtenus par copolymérisation de monomère(s) destiné(s) à former la matrice de ladite empreinte moléculaire avec au moins un monomère de formule générale (I) :

X-(R)ₙ-Y-G (I),

dans laquelle :
- X représente un groupe monovalent polymérisable,
- n est égal à 0 ou 1,
- R représente une chaîne hydrocarbonée en C₁-C₁₀, notamment en C₁-C₅, linéaire, ramifiée ou cyclique, saturée ou insaturée, éventuellement interrompue par un ou plusieurs hétéroatomes choisis parmi N, O et S, et optionnellement substituée,
- Y représente une fonction de liaison labile impliquant la fonction sulfhydryle du glutathion GSH ou de l'un de ses analogues,
- G représente le résidu du glutathion GSH ou de l'un de ses analogues correspondants,
dans des conditions propices à la formation de ladite empreinte moléculaire,
suivie de la libération du résidu G par clivage de la fonction de liaison Y.

Selon un mode de réalisation, X peut représenter un groupe monovalent choisi parmi les groupes vinyliques, acryliques, méthacryliques, allyliques, styréniques ou tout autre groupe insaturé susceptible de réagir par voie radicalaire, et les groupements chimiques permettant une réaction de polycondensation ou de sol-gel.

Il peut notamment s'agir d'un groupe polymérisable de type vinylique, acrylique ou méthacrylique, en particulier un groupe acrylique ou méthacrylique, et de préférence un groupe méthacrylique.

Selon un mode de réalisation, R peut représenter une chaîne hydrocarbonée linéaire en C₁-C₄, en particulier en C₂-C₄ et de préférence en C₂.

Selon un mode de réalisation, Y peut représenter une fonction disulfure -SS-.

Selon un mode de réalisation, G peut représenter un résidu d'un analogue du glutathion, de préférence le N-boc-glutathion.

A titre de monomère de formule générale (I), on peut notamment citer le méthacrylate du disulfure du N-boc-glutathion, qui est dérivé du méthacrylate du disulfure du glutathion, lui-même dérivé du glutathion.

Le polymère à empreintes moléculaires selon la présente invention est susceptible d'être obtenu par copolymérisation en outre d'au moins un autre monomère, notamment choisi parmi des agents de réticulation.

A titre d'agent de réticulation, on peut notamment citer p-divinylbenzène (DVB), 1,3 diisopropènyl benzène (DIP), diméthacrylate d'éthylène glycol (EGDMA), diméthacrylate de tétraméthylène (TDMA), *N*,*O*-bisacryloyl-L-phénylalaninol, 2,6-bisacryloylamidopyridine, 1,4-phénylène diacrylamide, *N*,*N*'-1,3-phénylènebis(2-méthyl-2-propenamide) (PDBMP), acide 3,5-bisacrylamido benzoïque, 1,4-diacryloyl pipérazine (DAP), N,N'-méthylène bisacrylamide (MDAA), N,N'-éthylène bisméthacrylamide, N,N'-tétraméthylène bisméthacrylamide, *N*,*N*'-hexaméthylène bisméthacrylamide, diméthacrylate d'anhydroerythritol, 1,4 ;3,6-dianhydro-D-sorbitol-2,5-diméthacrylate, isopropoylénébis(1,4-phénylène) diméthacrylate, triméthylpropane triméthacrylate (TRIM), pentaérythritol triacrylate (PETRA), pentaérythritol tétraacrylate (PETEA).

L'agent de réticulation est de préférence choisi parmi le diméthacrylate de l'éthylène glycol et le divinylbenzène.

La synthèse de l'empreinte moléculaire peut se faire par polymérisation en solution, en émulsion, en suspension, par précipitation, en microémulsion, par polymérisation en phase dispersée ou dans des conditions de préparation de microgels.

La matrice de l'empreinte moléculaire formée peut être de nature polyacrylates, polyméthacrylates, polyacrylamides, polyvinyliques, polyacryléine, polyacrylonitrile, poly(alcool vinylique), polyalkylvinylcétone, polybenzothiazole, poly carbonatede bis-phénol A, poly(chlorure de diallyl-diméthyl-ammonium), polychlorure de vinyle, polysiloxane, polyétheraromatique, polyéthersulfone, polyétherimide, polyéthylène imine, polyimide, polyimidazole, polyoxyméthylène, polyoxazole, polyoxyphénylène, polyoxytétraméthylène, polyvinylalkyléther, polyvinylpirrolidone et polyvinylméthylcétone.

Les MIPs susceptibles d'être obtenus par copolymérisation d'au moins un monomère de formule générale (I) telle que défini précédemment présentent une reconnaissance plus sélective pour le glutathion GSH et/ou pour ses analogues, que des MIPs obtenus suivant une approche classique. Cet aspect est notamment illustré par l'exemple 5 qui met en évidence l'importante différence de sélectivité entre un MIP synthétisé suivant l'approche classique et un MIP selon l'invention.

Par ailleurs, comme il ressort aussi de cet exemple comparatif ainsi que de l'exemple 6, les MIPs conformes à l'invention présentent en outre une reconnaissance plus sélective pour le glutathion GSH et/ou ses analogues que pour les adduits du GSH.

L'utilisation d'un monomère de formule générale (I) telle que défmie précédemment permet en effet de conférer à la cavité du MIP qui se forme lors de la polymérisation, une conformation interne proche de celle de la molécule de GSH ou de l'un de ses analogues ayant servi d'entité patron. Par suite, les adduits du GSH (qui sont démunis de la fonction sulfhydryle native du GSH et de ses analogues, étant donnée que c'est précisément cette fonction qui est impliquée dans leur formation) présenteront une interaction avec le MIP conforme à l'invention qui sera moins sélective que celle du GSH et/ou de l'un de ses analogues avec ce même MIP.

### PROCEDE

Comme indiqué précédemment, l'invention concerne également un procédé de traitement d'un milieu susceptible de comprendre, ou comprenant, la forme libre du glutathion GSH et/ou l'un des ses analogues et au moins un ou plusieurs adduits du GSH, comprenant au moins :
(a) une étape de mise en contact dudit milieu avec au moins un polymère à empreintes moléculaires conforme à l'invention dans des conditions propices à l'extraction du glutathion GSH et/ou de ses analogues, si présents, par ladite empreinte moléculaire,
(b) la séparation dudit polymère à empreintes moléculaires du milieu ainsi traité, et
(c) le cas échéant, la libération du glutathion GSH et/ou de ses analogues dudit polymère à empreintes moléculaires résultant de l'étape (b).

A l'issue de l'étape (b), on obtient donc un milieu purifié, c'est-à-dire significativement appauvri, voir dénué de GSH et/ou de l'un de ses analogues.

Pourront demeurer, le cas échéant, dans le milieu purifié les entités n'ayant aucune interaction avec le MIP, et par exemple le candidat médicament.

Selon un mode de réalisation, l'étape (b) peut être suivie d'une libération spécifique du GSH et/ou de ses analogues.

Selon un mode de réalisation particulier de l'invention, le milieu traité peut être un milieu d'incubation d'une molécule active mise en oeuvre dans un test de toxicologie au glutathion, par exemple tel que défini précédemment.

Le procédé précédent selon l'invention peut être suivi d'une analyse qualitative et/ou quantitative du milieu traité obtenu à l'issue de l'étape (b) pour la caractérisation des adduits du GSH selon toute méthode connue de l'homme du métier.

Selon une variante de réalisation, le procédé selon l'invention peut comprendre en outre, préliminairement à l'étape (a), au moins :
(i) une étape de mise en contact d'un milieu, dit complexe, susceptible de comprendre, outre du glutathion GSH et/ou l'un de ses analogues, au moins un ou plusieurs adduit(s) du GSH, avec au moins un premier polymère à empreintes moléculaires apte à interagir avec les adduits du GSH, le glutathion et ses analogues, dans des conditions adaptées à l'extraction des adduits du GSH, du glutathion et de ses analogues,
(ii) la séparation dudit polymère à empreintes moléculaires du milieu ainsi traité, et
(iii) la formation d'un milieu enrichi en lesdits adduits du GSH, en ledit glutathion et en sesdits analogues, par libération des adduits du GSH, du glutathion et de ses analogues du polymère à empreintes moléculaires résultant de l'étape (ii).

Ce milieu peut subir ensuite l'étape (a) du procédé selon l'invention.

Les MIPs peuvent être mis en oeuvre sur tout support approprié.

Par « support », s'entend très largement, au sens de l'invention, tout substrat solide, flexible ou rigide, sur ou dans lequel les MIPs sont susceptibles d'être liés, collés, déposés, synthétisés in-situ, remplis et/ ou conditionnés.

Les supports utilisables selon l'invention peuvent être de toute nature, comme par exemple de nature biologique, non biologique, organique, inorganique, ou encore une de leur combinaison. Ils peuvent se présenter sous toute forme, et notamment prendre la forme de particules, de gels, de feuilles, de tubes, de sphères, de capillaires, de points, de films, de puits, de toute taille et de toute forme.

Ils peuvent par exemple se présenter sous la forme de particules de taille homogène, notamment comprise entre 10 nm et 10 mm, de préférence entre 25 et 45 µm susceptibles d'être par suite conditionnées sous forme de cartouche.

De manière générale, les MIPs peuvent par exemple être mis en oeuvre sur ou dans un support choisi parmi une cartouche SPE, une plaque multipuits, comme par exemple une plaque à 96 puits, un patch, un sachet de thé (« tea bag » en langue anglaise), un microtube, une colonne HPLC, une bandelette, des puces, des lames, des plaques de silice, des couches minces, une surface poreuse, une surface non poreuse, un système microfluidique.

Selon un mode de réalisation de l'invention, les polymères à empreintes moléculaires peuvent être mis en oeuvre sur une colonne d'extraction, par exemple une cartouche SPE.

Ainsi, selon un mode de réalisation de l'invention, le procédé peut comprendre au moins une étape d'extraction en phase solide (SPE).

Une procédure d'extraction en phase solide comporte généralement trois ou quatre étapes. La première est le conditionnement de l'adsorbant contenu dans la cartouche d'extraction, qui permet de mouiller le support en solvatant les groupements fonctionnels présents à sa surface. Lors de la seconde étape, on procède à la percolation de la solution à traiter sur le MIP, de sorte à ce que les entités n'ayant aucune affmité avec ce dernier ne soient pas retenues. En revanche, la (ou les) molécule(s) cible(s), et éventuellement d'autres entités présentant une forte affinité avec l'adsorbant, restent sur le support à l'issue de cette étape.

Une étape supplémentaire de lavage peut être effectuée afin d'éliminer les entités faiblement retenues (le candidat médicament par exemple) par le support, au moyen d'un solvant de force éluante convenable pour éluer ces entités tout en gardant la (ou les) molécule(s) cible(s) sur le support.

Si nécessaire, on peut procéder enfin à l'élution de la (ou des) molécule(s) cible(s) par passage d'un solvant spécifiquement choisi pour permettre de rompre les interactions de reconnaissance mises enjeu entre la (ou les) molécule(s) cible(s) et le MIP tout en évitant d'éluer des entités interférentes fortement retenues sur le support, de telle sorte à libérer la (ou les) molécule(s) cible(s) extraite(s).

A la fin de ce processus d'extraction et de libération, on peut donc obtenir une solution purifiée, et éventuellement enrichie en molécule(s) cible(s).

Par exemple, dans le cas d'une application du procédé de l'invention à des fins de traiter un milieu mis en oeuvre dans un test de toxicologie au glutathion, l'intérêt est de retenir les molécules cibles du MIP conforme à l'invention (à savoir le glutathion GSH et/ou ses analogues) sur le support et d'obtenir une solution éluée comprenant les adduits du GSH (retenus moins sélectivement sur le support) et appauvrie en glutathion GSH et/ou en ses analogues.

Typiquement, les solvants utilisés lors d'une extraction en phase solide peuvent être des solvants organiques comme par exemple l'acétonitrile, le méthanol, le dichlorométhane, des solvants aqueux comme par exemple l'eau, des solutions tampons, les solvants pouvant être utilisés en mélange et avec différentes conditions de salinité, de pH, de polarité.

D'autres types d'étapes de prétraitement peuvent être envisagées comme par exemple la micro-extraction en phase solide (SPME ou « solid phase micro-extraction » en langue anglaise), l'extraction dynamique sur phase solide (SPDE ou « solid phase dynamic extraction » en langue anglaise), l'extraction sur barreaux d'agitation (SBSE ou « Stir Bar Sorption Extraction » en langue anglaise), des capillaires, des bandes, des puces.

Selon une variante de réalisation, avant sa mise en oeuvre dans le milieu à traiter, la cavité du MIP conforme à l'invention pourra contenir une molécule attachée de façon covalente ou non covalente distincte du GSH ou de l'un de ses analogues, par exemple un marqueur, et étant destinée à être déplacée par le GSH et/ou de l'un de ses analogues lors de l'interaction de celui-ci avec son site de reconnaissance.

Le marqueur peut notamment être détecté, par exemple après relargage, par colorimétrie visible comme par exemple à l'oeil nu, par radiochimie, par médecine nucléaire comme par exemple par scintigraphie, par imagerie, par résonance (IRM), par rayons X, par diffusion de lumière, par spectrométrie de masse, par spectroscopie, comme par exemple par fluorescence ou par UV-visible, par ultrasons, par radioactivité, par réfractométrie, par détections optique, piézoélectrique, acoustique, par électrochimie, par conductivité, par pH métrie, ou encore par voie biologique, et de préférence par l'oeil nu.

Les exemples figurant ci-après sont présents à titre illustratif et non limitatif du domaine de l'invention.

### EXEMPLES

Deux MIPs reconnaissant le GSH ont été préparés, l'un par une approche classique et l'autre par une approche semi covalente, et leurs propriétés de reconnaissance vis-à-vis du GSH ont été mises en évidence.

### Exemple 1 : Synthèse de polymères à empreintes moléculaires suivant l'approche « classique » non covalente

### a) Synthèse de la molécule cible N-BOC-GS-Ac

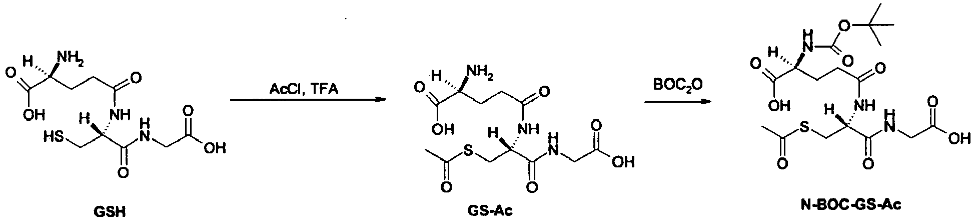

### γ-L-glutamyl-S-acetyl-L-cysteinyl-glycine (GS-Ac).

Dans un ballon surmonté d'un réfrigérant, 5 g de glutathion (16,3 mmol) sont dissous dans 55 mL d'acide trifluoroacétique. On met le montage sous azote et on fait buller de l'azote dans la solution pendant 5 minutes. Une fois le ballon mis sous azote, on additionne 2 mL d'acide acétique. On porte à 40 °C pendant 20 minutes. A température ambiante, on additionne 1,5 mL d'eau puis on chauffe à 40 °C pendant 20 minutes. On évapore ensuite à la pompe puis on ajoute de l'acétate d'éthyle. Le produit précipite sous forme de pâte collante. Le produit est alors solubilisé dans le minimum d'eau chaude puis on ajoute de l'acétone. On met à 4 °C pendant 3 jours. Le produit blanc précipité est filtré. 3,38 g de produit attendu sont récupérés.
Rendement : 59 %
**RMN ¹H** (DMSO-d₆, 300MHz) δ 1,82 (m, 2H, CH₂(Glu)), 2,32 (m, 5H, Me et CH₂-C(O) (Glu), 2,96 (dd, J=9,4 et 13,4 Hz, CHS, 1H), 3,33-3,39 (dd et t, 2H, CHS et CHNC(O) (Glu)), 3,70 (d, J=5,46 Hz, 2H, N-CH₂-C(O) (Gly)), 4,37-4,45 (m, 1H, CHN(Cys)), 8,51 (d, J=8,5 Hz, NH (Cys), 1H), 8,64 (t,J=5,6 Hz, NH (Gly), 1H).
**SM-ESI** (mode négatif) = 348,27 ([M-H]⁻)

### γ-L-N-BOC-glutamyl-S-acetyl-L-cysteinyl-glycine (N-BOC-GS-Ac)

2 g de γ-*L*-glutamyl-S-acetyl-*L*-cysteinyl-glycine (5,76 mmol) sont mis en suspension dans 13 mL de dioxane. On ajoute 13 mL d'eau puis goutte à goutte une solution aqueuse de K₂CO₃ (791 mg, 1 éq. dans 13 mL d'eau). Une effervescence se produit. On ajoute à 0 °C le BOC₂O dissous dans 2 mL de dioxane. La solution se trouble puis devient limpide. On agite 2 heures à température ambiante. Le milieu est évaporé puis on ajoute de l'acétate d'éthyle. Une solution aqueuse à 4 % de NaHSO₄ dans l'eau est additionnée de façon à être entre pH 2 et pH 3. On extrait trois fois la phase aqueuse avec de l'acétate d'éthyle. La phase organique est alors lavée à l'eau et elle est ensuite séchée sur MgSO₄. 1,2 g d'un produit blanc est obtenu.
Rendement : 46 %.
**RMN ¹H** (CD₃CN, 300MHz) δ1,33 (s, 9H, tBu), 1,76-2,02 (m, 2H, CH₂(Glu)), 2,24 (m, 5H, Me et CH₂-C(O) (Glu), 3,03 (dd, J=8.1 et 13,92 Hz, CHS, 1H), 3,29 (dd, J=4,89 et 13,95 Hz, CHS, 1H), 3,81 (d, J=5,85 Hz, 2H, N-CH₂-C(O) (Gly)), 4,03 (m, 1H, CHN), 4,46 (m, 1H, CHN), 5,79 (d, J=7,74 Hz, NH(BOC), 1H), 6,99 (d, J=7,92 Hz, NH (Cys), 1H), 7,18 (t, J=5.64 Hz, NH (Gly), 1H).
**SM-ESI** (mode négatif) = 448,40 ([M-H]⁻)

### b) Synthèse des matériaux imprimé et non imprimé n°1 correspondants (approche classique)

Le diméthylacrylate de l'éthylène glycol est lavé plusieurs fois par une solution basique saturée au NaCl pour éliminer l'inhibiteur. Il est séché sur MgSO₄. L'amorceur azobisisobutyronitrile (AIBN) est recristallisé dans l'acétone.

Le matériau imprimé (empreinte) n° 1 est préparé en mélangeant 279 mg de N-BOC-GS-Ac, 4,9 g de diméthylacrylate de l'éthylène glycol, 551 mg d'acrylate de 2-carboxyéthyle et 131 mg de 4-vinylpyridine dans 6,9 mL d'acétonitrile anhydre. Le mélange est dégazé en faisant buller 10 minutes de l'azote puis on ajoute 57 mg d'AIBN. La polymérisation s'effectue à 50 °C pendant 72 heures pour former un monolithe coloré.

Le matériau non imprimé n° 1 est préparé en mélangeant 5,0 g de diméthylacrylate de l'éthylène glycol, 524 mg d'acrylate de 2-carboxyéthyle et 143 mg de 4-vinylpyridine dans 6,9 mL d'acétonitrile anhydre. Le mélange est dégazé en faisant buller 10 minutes de l'azote puis on ajoute 57 mg d'AIBN. La polymérisation s'effectue à 50 °C pendant 72 heures pour former un monolithe coloré.

Les matrices préparées ci-dessus sont broyées puis tamisées. Les particules dont la taille est située entre 25 et 45 µm sont introduites dans une colonne HPLC 150 x 4.6 mm puis tassées par pression et lavées avec un mélange 5 % Acide Acétique dans Acétonitrile/H₂O (97,5/2,5) puis de l'acétonitrile pour l'étude de la reconnaissance en HPLC. On dispose ainsi de 2 colonnes HPLC.

### Exemple 2: Synthèse de polymères à empreintes moléculaires suivant l'approche semi-covalente

### a) Synthèse d'un monomère dérivé du glutathion

Un monomère nommé méthacrylate du disulfure du N-Boc glutathion a été synthétisé en 4 étapes, avec un rendement global de 13 %.

La première étape est décrite par plusieurs auteurs, et notamment Lapeyre *et al.* La deuxième étape est décrite par Bulmus *et al* avec un rendement de 35 % en masse ; le mode opératoire a été légèrement modifié, et le produit a été obtenu avec un rendement de 63 %. La troisième étape a permis d'obtenir de manière quantitative le monomère dérivé du glutathion. La dernière étape est la protection de la fonction amine par un groupement
t-butylcarbamate.

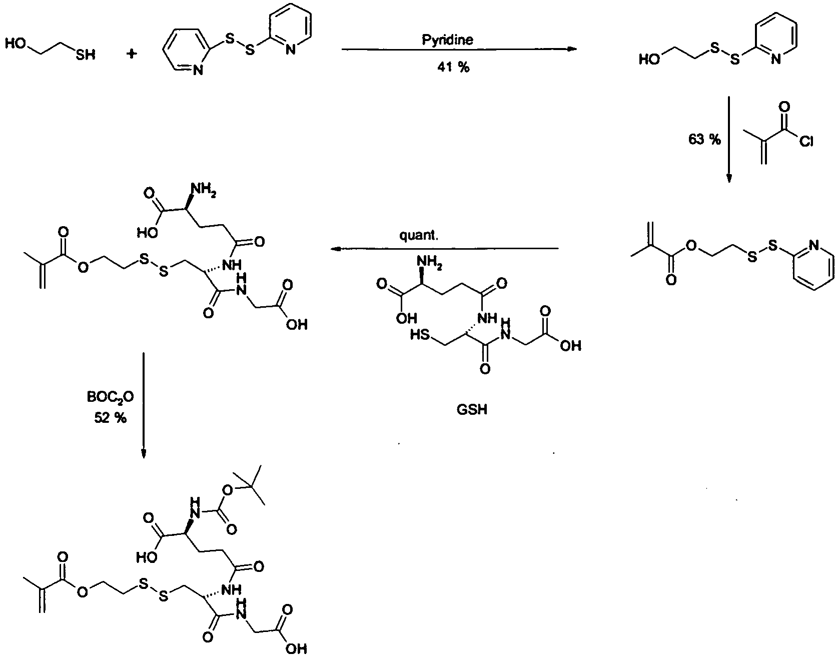

### 2-Pyridin-2-yldisulfanyl-éthanol.

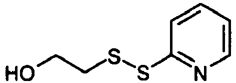

2.25 g de bis(2pyridyl)disulfide sont dissous dans 100 mL de pyridine:méthanol (1:99). Le β-mercaptoéthanol (700 µL, 1 éq.) est ajouté goutte à goutte à la solution. On observe une couleur jaune immédiate. Le milieu est agité pendant une nuit. Le lendemain on n'observe plus de produit de départ et on évapore la solution. Le résidu est passé sur deux colonnes de silice, pour obtenir 770 mg du produit attendu. (colonne réalisée au dichlorométhane avec un léger gradient d'acétate d'éthyle) Seules les fractions les plus propres sont évaporées.
Rendement : 41 %. (huile incolore)
**Rf** = 0,48 (DCM/AcOEt 80/20)
**RMN ¹H** (CDCl₃, 300 MHz) δ 2,83 (t, J=5,46 Hz, 2H, CH₂S), 3,69 (t, J=5,25 Hz, 2H, CH₂O), 5,42 (bs,OH,1H), 6,99-7,04 (m,1H), 7,36 (m,1H), 7,45-7,51 (m,1H), 8,34 (m,1H).

### Méthacrylate du disulfure de pyridyle.

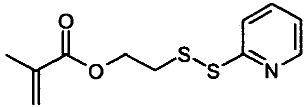

Dans 50 mL de DCM on additionne le disulfure (770 mg) puis la triethylamine (860 µL, 1,5 éq.). A 0 °C on ajoute le chlorure de méthacryloyle (400 µL, 1,2 éq.) goutte à goutte. La solution est agitée à 0°C pendant 30 minutes. La solution est agitée à température ambiante pendant une nuit. La phase organique est lavée trois fois avec de l'eau et trois fois avec une solution saturée de NaHCO₃. La phase organique est séchée sur MgSO₄ et est évaporée. Le résidu est passée sur une colonne de silice. La colonne est packée au cyclohexane et une fois le résidu déposé on élue avec du dichlorométhane. On récupère une fraction du produit attendu (660 mg)
Rendement : 63 %.
**Rf** = 0,84 (DCM/AcOEt 80/20)
**RMN ¹H** (CDCl₃, 300 MHz) δ 1,82 (s, Me, 3H), 2,99 (t, J=6,36 Hz, 2H, CH₂S), 4,28 (t, J=6,36 Hz, 2H, CH₂O), 5,47 (s, CH=, 1H), 6,01 (s, CH=, 1H), 6,98 (m,1H), 7,49-7,61 (m,2H), 8,34 (d, J=4,8 Hz, 1H).
**RMN ¹³C** (CDCl₃, 75 MHz) δ 18,54 (Me), 37,64 (SCH₂), 62,63 (OCH₂), 119,91 (CH), 121,14 (CH), 126,31 (=CH₂), 136,16 (C^{IV}), 137,39 (CH), 149,91 (CH), 159,95 (C^{IV}), 167,15 (C^{IV}).
**Conditions HPLC n°1** : t_{R} = 15.98 min. ; t_{R} (pyridylthione)= 5.03 min. ; t_{R} (GSH)= 3.7 min.
**Conditions HPLC n°1** : colonne Hypersil Gold 100 x 4.6 mm. Injection de 5 µL. 1mL/min. Gradient : 0.1 % TFA dans l'eau pendant 2 minutes puis 80/20 ACN/0.1 % TFA Eau jusqu'à 20 minutes puis constant jusqu'à 25 minutes

### Monomère méthacrylate du disulfure du glutathion.

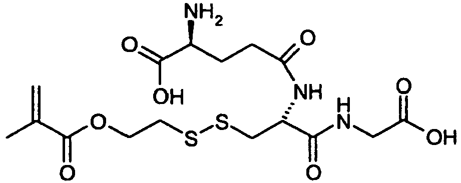

820 mg du monomère méthacrylate de disulfure de pyridyle sont mis en solution dans 10 mL ACN puis 2.6 mL de DMF puis 15 mL d'eau. On ajoute 1,1 équivalent de GSH (1,09 g). Très rapidement on observe la coloration de la solution à cause de la libération de la pyridylthione. Après 5 minutes sous agitation, la solution est diluée dans le même volume d'eau puis lyophilisée pour la nuit. On obtient alors une poudre jaune claire. On lave la poudre avec 4 fois 50 mL d'acétonitrile jusqu'à ce que le filtrat soit incolore. On obtient alors une poudre blanche qui est séchée sous vide. 1.48 g de produit est obtenu.
Rendement quantitatif.
**SM-ESI** (mode positif) = 452,07 ([M-H]+), 902,8 ([2M+H)⁺])
**Conditions HPLC n°1 :** t_{R} = 10,85 min. ; t_{R} (pyridylthione)= 5,03 min. ; t_{R} (GSH)= 3,7 min.

### Monomère méthacrylate du disulfure du N-Boc glutathion.

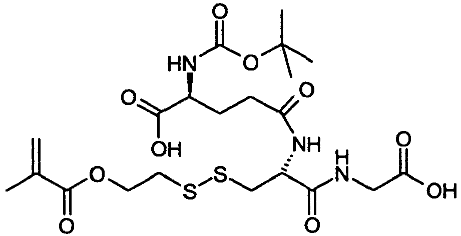

500 mg du monomère méthacrylate de disulfure du glutathion sont mis en solution dans 2 mL de dioxane, 1 mL d'eau et 306 mg de K₂CO₃ dilués dans 2 mL d'eau. A la suspension on ajoute 4 mL d'eau et 3 mL de dioxane. A 0 °C on ajoute 1,1 équivalent de BOC₂O. On laisse sous agitation à température ambiante pendant 30 minutes. Le milieu est évaporé sous vide. On rajoute de l'acétate d'éthyle et on acidifie à froid avec une solution aqueuse à 4 % de KHSO₄ jusqu'à pH 2. On extrait avec de l'acétate d'éthyle 2 fois. 351 mg d'une huile légèrement jaune sont obtenus.
Rendement : 52 %.
**Conditions HPLC n°1 :** t_{R} = 19,1 min. ; t_{R} (pyridylthione)= 5,03 min. ; t_{R} (GSH)= 3,7 min.
**SM-ESI** (mode négatif) = 550,07 ([M-H]⁻)

### b) Synthèse des matériaux imprimés et non imprimés n° 2 correspondants (approche semicovalente)

Le diméthylacrylate de l'éthylène glycol est lavé plusieurs fois par une solution basique saturée au NaCl pour éliminer l'inhibiteur. Il est séché sur MgSO₄. L'amorceur azobisisobutyronitrile (AIBN) est recristallisé dans l'acétone.

Le matériau imprimé (empreinte) n° 2 est préparé en mélangeant 344 mg de monomère méthacrylate du disulfure du N-Boc glutathion, 4,96 g de diméthylacrylate de l'éthylène glycol, 540 mg d'acrylate de 2-carboxyéthyle et 131 mg de 4-vinylpyridine dans 6,9 mL d'acétonitrile anhydre. Le mélange est dégazé en faisant buller 10 minutes de l'azote puis on ajoute 57 mg d'AIBN. La polymérisation s'effectue à 50 °C pendant 72 heures pour former un monolithe coloré.

Le matériau non imprimé n° 2 est préparé en mélangeant 5,0 g de diméthylacrylate de l'éthylène glycol, 540 mg d'acrylate de 2-carboxyéthyle, 81 mg du méthacrylate du 2-hydroxyéthyle et 131 mg de 4-vinylpyridine dans 6,9 mL d'acétonitrile anhydre. Le mélange est dégazé en faisant buller 10 minutes de l'azote puis on ajoute 57 mg d'AIBN. La polymérisation s'effectue à 50 °C pendant 72 heures pour former un monolithe coloré.

Les matrices préparées ci-dessus sont broyées puis tamisées. Les particules dont la taille est située entre 25 et 45 µm sont introduites dans une colonne HPLC 150 x 4,6 mm puis tassées par pression. Les deux colonnes sont mises en série et en circuit fermé dans une solution 50/50 MeCN/ Tampon phosphate pH=10 (solution Merck) contenant 300 mg de DTT pendant 48 heures. Les colonnes sont ensuite lavées avec de l'eau puis avec de ACN, pour l'étude de la reconnaissance en HPLC. Ainsi on dispose de 2 colonnes HPLC.

### Exemple 3 : Evaluation de la reconnaissance du matériau n° 1 (approche classique)

Deux solutions à 2 mM et 5mM de glutathion dans l'eau sont injectées sur les deux colonnes remplies respectivement de l'empreinte n° 1 et du matériau non imprimé n° 1.

L'éluant utilisé est un mélange à 90/10 de Acétonitrile/tampon 10 mM à différents pH avec un débit de 1 mL/min. La détection du glutathion est faite avec un détecteur à diffusion de lumière (DEDL). Les volumes d'injection sont de 20 µL.

On détermine les valeurs de k' (facteur de capacité) et d'IF (facteur d'empreinte) pour évaluer la reconnaissance du glutathion sur les matrices.

| **Analyte** | **k'_{MIP n°1}** | **k'_{matériau non imprimé n°1}** | **IF** |
|---|---|---|---|
| Glutathion 2 mM | 5,48 | 1,52 | 3,60 |
| Glutathion 5 mM | 5,58 | 1,50 | 3,71 |

| | | | |
|---|---|---|---|
| *Conditions HPLC : Glutathion, éluant : 90*/*101 % AA ACN*/*H₂O, particules 25-45* µ*m.* | | | |

Dans les conditions d'analyse utilisées on observe une reconnaissance de l'empreinte n° 1 importante pour le glutathion à des concentrations élevées.

### Exemple 4 : Evaluation de la reconnaissance du matériau n°2 (approche semi-covalente)

Deux solutions à 4,5 mM et à 2,2 mM de glutathion dans une solution 85/15 MeCN/Eau sont injectées sur les deux colonnes remplies respectivement de l'empreinte n° 2 et du matériau non imprimé n° 2.

L'éluant utilisé est un mélange à Acétonitrile/Eau avec différents pourcentages et en présence ou non d'acide acétique avec un débit de 1 mL/min. La détection du glutathion est faite avec un détecteur à diffusion de lumière (DEDL). Les volumes d'injection sont de 20 µL.

On détermine les valeurs de k' (facteur de capacité) et d'IF (facteur d'empreinte) pour évaluer la reconnaissance du glutathion sur les matrices.

| | 90/10 (1 % AA ACN/H₂O) | | 90/10 (0.5 % AA ACN/H₂O) | |
|---|---|---|---|---|
| | Empreinte n°2 | Matériau non imprimé n°2 | Empreinte n°2 | Matériau non imprimé n°2 |
| GSH 4.5 mM | 12,776 | 4,643 | 17,149 | 5,735 |

| **Analyte** | **k'_{MIP n°1}** | | **k'_{matériau non imprimé n°1}** | **IF** |
|---|---|---|---|---|
| Glutathion 4.5 mM | 9,09 | | 2,19 | 4,16 |

| | | | | |
|---|---|---|---|---|
| *Conditions HPLC : éluant : 90*/*10 0.5 % AA ACN*/*H₂O, particules 25-45 µm.* | | | | |

Dans les conditions d'analyse utilisées on observe une reconnaissance de l'empreinte n° 2 importante pour le glutathion à des concentrations élevées. Cette reconnaissance est plus importante que celle de l'empreinte n° 1.

### Exemple 5 : Comparaison

Différentes solutions de GSH et d'adduits du GSH dans une solution 85/15 MeCN/Eau sont injectées sur les deux colonnes remplies respectivement de l'empreinte n° 1 et de l'empreinte n° 2.

L'éluant utilisé est un mélange à 1 % Acide Acétique Acétonitrile/Eau avec un ratio de 90/10 avec un débit de 1 mL/min. La détection du glutathion et des adduits est faite avec un DEDL. Les volumes d'injection sont de 20 µL.

On détermine les valeurs de k' (facteur de capacité) et de sélectivité pour évaluer la reconnaissance du glutathion GSH et des adduits du GSH sur les empreintes.

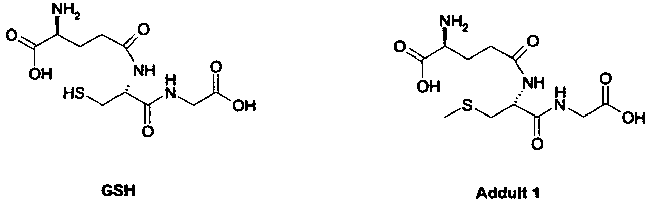

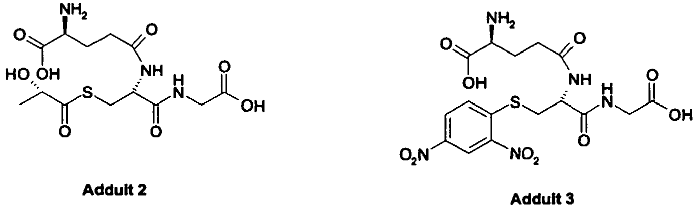

| | 90/10 (1 % AA ACN/H₂O) ; Empreinte n° 1 | | | |
|---|---|---|---|---|
| | Temps de rétention | Largeur à mi-hauteur | k' | Sélectivité |
| GSH 5,07 mM | 9,213 | 1,8 | 4,42 | 100 |
| GSH 2 mM | 9,078 | 1,7 | 4,34 | 98 |
| Adduit 1 5,91 mM | 7,693 | 3,2 | 3,55 | 80 |
| Adduit 2 4,48 mM | 9,823 | 2,2 | 4,78 | 108 |

| | 90/10 (1 % AA ACN/H₂O); Empreinte n°2 | | | |
|---|---|---|---|---|
| | Temps de rétention | Largeur à mi-hauteur | k' | Sélectivité |
| GSH 2,3 mM | 11,214 | 4,5 | 7,01 | 100 |
| Adduit 1 3,7 mM | 7,406 | 1,5 | 4,29 | 61 |
| Adduit 2 2,4 mM | 7,893 | 1,6 | 4,64 | 66 |

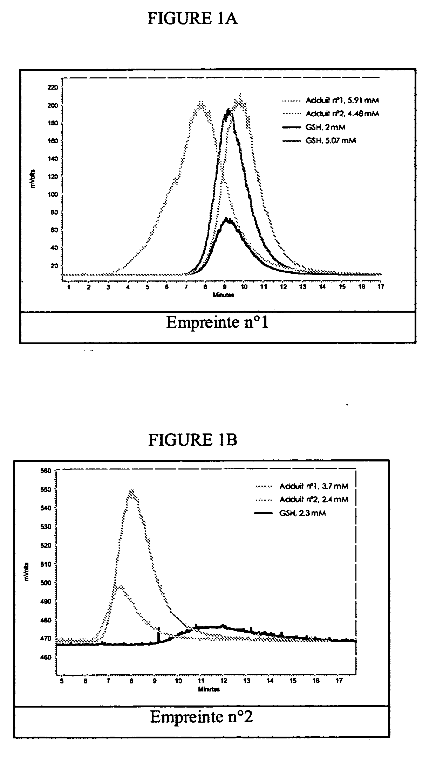

Ces résultats sont respectivement illustrés par les figures 1A et 1B.

Lors de l'injection du glutathion sur les deux empreintes, le glutathion sort plus rapidement sur l'empreinte n° 1 et la largeur du pic est moins importante. Par ailleurs, l'empreinte n° 1 est peu sélective par rapport aux adduits.

De plus, on constate que l'empreinte n° 2 est plus sélective du glutathion que l'empreinte n° 1.

### Exemple 6 : Reconnaissance et sélectivité de l'empreinte n° 2 vis-à-vis des adduits du GSH par SPE

Une cartouche de SPE est réalisée en introduisant 200 mg de l'empreinte n° 2 entre deux frittés. Préalablement à l'extraction, 5 mL d'acétonitrile et 6 mL d'une solution à 90/10 ACN (1 % AA)/H₂O sont passés sur la cartouche pour la conditionner avant d'introduire la solution à percoler. Puis 1 mL d'une solution contenant 182,89 µg de GSH (0,60 mM), 5,8 µg d'adduit 2 (0,015 mM) et 4,14 µg d'adduit 3 (0,009 mM) dans 90/10 MeCN (1 %AA)/Eau est percolé sur la cartouche de SPE. Plusieurs fractions de 500 µL d'une solution à 90/10 MeCN (1 %AA)/Eau sont utilisées comme solution de lavage. Par la suite 3 mL d'une solution 85/15 ACN/H₂O sont percolés. Les diverses fractions sont ensuite analysées par HPLC-UV.

Le tableau suivant donne les taux de récupération (%) en adduits 2 et 3 et en GSH obtenus lors de cette extraction.

| Fraction | Taux de récupération % en Adduit 3 | Taux de récupération % en Adduit 2 | Taux de récupération % en GSH |
|---|---|---|---|
| Percolation | 0 | 0 | 0 |
| Lavage 1 | 0 | 0 | 0 |
| Lavage 2 | 0 | 0 | 0 |
| Lavage 3 | 0 | 30 | 0 |
| Lavage 4 | 29 | 42 | 3 |
| Lavage 5 | 33 | 20 | 8 |
| Lavage 6 | 21 | 8 | 12 |
| 3mLACN/H₂O 85/15 | 13 | 0 | 72 |

On observe dans ces conditions d'utilisation une différence de comportement de la matrice de l'empreinte n° 2 vis-à vis des adduits du GSH et du GSH.

Les adduits ne sont pas retenus dans les solutions de lavage et une grande partie du glutathion (72 %) a pu être éliminée de l'échantillon.

### Conclusion

Le MIP préparé par une approche classique présente une reconnaissance à la fois pour le GSH et pour ses adduits tandis que le MIP préparé par une approche semi covalente présente une reconnaissance plus importante pour le GSH, et donc une meilleure sélectivité.

Le MIP obtenu à partir d'une approche semi covalente peut permettre de réaliser une étape de pré-traitement avant l'analyse des milieux d'incubation. Cette étape de pré-traitement permet d'éliminer sélectivement le GSH, tout en conservant dans la solution pré-traitée les adduits du GSH et procéder ainsi à une purification, et éventuellement à un enrichissement, en adduits du GSH.

## Revendications

1. Procédé de préparation d'un polymère à empreintes moléculaires (MIP) dédié à la reconnaissance sélective de la forme réduite du glutathion, GSH, ou analogue, comprenant au moins :
- la copolymérisation de monomère(s) destiné(s) à former la matrice de ladite empreinte moléculaire avec au moins un monomère de formule générale (I) :
X-(R)ₙ-Y-G (I),
dans laquelle :
- X représente un groupe monovalent polymérisable,
- n est égal à 0 ou 1,
- R représente une chaîne hydrocarbonée en C₁-C₁₀, notamment en C₁-C₅, linéaire, ramifiée ou cyclique, saturée ou insaturée, éventuellement interrompue par un ou plusieurs hétéroatomes choisis parmi N, O et S, et optionnellement substituée,
- Y représente une fonction de liaison labile impliquant la fonction sulfhydryle du glutathion GSH ou de l'un de ses analogues,
- G représente le résidu du glutathion GSH ou de l'un de ses analogues correspondants,
dans des conditions propices à la formation de ladite empreinte moléculaire, et
- la libération du résidu G par clivage de la fonction de liaison Y.

2. Procédé selon la revendication 1, **caractérisé en ce que** X représente un groupe monovalent choisi parmi les groupes vinyliques, acryliques, méthacryliques, allyliques, styréniques ou tout autre groupe insaturé susceptible de réagir par voie radicalaire, et les groupements chimiques permettant une réaction de polycondensation ou de sol-gel.

3. Procédé selon l'une quelconque des revendications précédentes, **caractérisé en ce que** Y représente une fonction disulfure -SS-.

4. Procédé selon l'une quelconque des revendications précédentes, **caractérisé en ce qu'**au moins un monomère de formule générale (I) est le méthacrylate du disulfure du N-boc-glutathion.

5. Polymère à empreintes moléculaires (MIP) susceptible d'être obtenu par le procédé selon l'une quelconque des revendications précédentes.

6. Procédé de traitement d'un milieu susceptible de comprendre, ou comprenant, au moins du glutathion GSH et/ou l'un de ses analogues, comprenant au moins :
(a) une étape de mise en contact dudit milieu avec au moins un polymère à empreintes moléculaires obtenu selon la revendication 5 dans des conditions propices à l'extraction du glutathion GSH et/ou de ses analogues, si présents, par ladite empreinte moléculaire,
(b) la séparation dudit polymère à empreintes moléculaires du milieu ainsi traité, et
(c) le cas échéant, la libération du glutathion GSH et/ou de ses analogues dudit polymère à empreintes moléculaires résultant de l'étape (b).

7. Procédé selon la revendication précédente, **caractérisé en ce que** le polymère à empreintes moléculaires est mis en oeuvre sur une colonne d'extraction, par exemple une cartouche SPE.

8. Procédé selon la revendication 6 ou 7, dans lequel ledit milieu est un milieu complexe susceptible de comprendre, outre du glutathion GSH et/ou l'un de ses analogues, au moins un ou plusieurs adduit(s) du GSH.

9. Procédé selon la revendication 8, **caractérisé en ce qu'**il comprend en outre, préliminairement à l'étape (a), au moins :
(i) une étape de mise en contact dudit milieu avec au moins un premier polymère à empreintes moléculaires apte à interagir avec les adduits du GSH, le glutathion et ses analogues, dans des conditions adaptées à l'extraction des adduits du GSH, du glutathion et de ses analogues,
(ii) la séparation dudit polymère à empreintes moléculaires du milieu ainsi traité, et
(iii) la formation d'un milieu enrichi en lesdits adduits du GSH, en ledit glutathion et en sesdits analogues, par libération des adduits du GSH, du glutathion et de ses analogues du polymère à empreintes moléculaires résultant de l'étape (ii).

10. Procédé selon l'une quelconque des revendication 8 ou 9, dans lequel le milieu complexe est un milieu d'incubation d'une molécule active mise en oeuvre dans un test de toxicologie au glutathion, comme par exemple un milieux d'incubation d'un candidat-médicament.

11. Utilisation d'au moins un polymère à empreintes moléculaires obtenu selon la revendication 5 des fins d'extraction, de détection, de séparation, de purification, d'absorption, d'adsorption, de rétention ou de libération contrôlée de la forme libre de glutathion GSH et/ou de l'un de ses analogues.

12. Utilisation d'au moins un polymère à empreintes moléculaires obtenu selon la revendication 5 pour le traitement d'un milieu susceptible de comprendre, ou comprenant, au moins du glutathion GSH et/ou l'un de ses analogues et des adduits du GSH.

## Claims

1. A method for preparing a molecularly imprinted polymer (MIP) dedicated to the selective recognition of the reduced form of glutathione, GSH, or analog, comprising at least:
- the copolymerization of a monomer or monomers intended to form the matrix of said molecular imprint with at least one monomer of general formule (I):
X-(R)ₙ-Y-G (I),
in which:
- X represents a polymerizable monovalent group,
- n is equal to 0 or 1,
- R represents a saturated or unsaturated, linear, branched or cyclic C₁-C₁₀, in particular C₁-C₅, hydrocarbon-based chain, optionally interrupted with one or more heteroatoms chosen from N, O and S, and optionally substituted,
- Y represents a function of a labile bond involving the sulfhydryl function of glutathione GSH or of an analog thereof,
- G represents the residue of glutathion, GSH or of a corresponding analog thereof,
under conditions favorable to the formation of said molecular imprint, and
- the release of the residue G by cleavage of the bond function Y.

2. The method as claimed in claim 1, **characterized in that** X represents a monovalent group chosen from vinyl, acrylic, methacrylic, allyl or styrene groups or any other unsaturated group capable of reacting via the free-radical process, and chemical groups enabling a polycondensation or sol-gel reaction.

3. The method as claimed in either one of the preceding claims, **characterized in that** Y represents a disulfide function -SS- .

4. The method as claimed in any one of the preceding claims, **characterized in that** at least one monomer of general formule (I) is N-boc-glutathione disulfide methacrylate.

5. A molecularly imprinted polymer (MIP) that can be obtained by means of the method as claimed in any one of the preceding claims.

6. A method for treating a medium that may comprise, or comprises, at least glutathione GSH and/or an analog thereof, comprising at least:
(a) a step of bringing said medium into contact with at least one molecularly imprinted polymer obtained according to claim 5 under conditions favorable to the extraction of the glutathione GSH and/or of analogs thereof, if present, by said molecular imprint,
(b) separation of said molecularly imprinted polymer from the medium thus treated, and
(c) where appropriate, release of the glutathione GSH and/or of analogs thereof from said molecularly imprinted polymer resulting from step (b).

7. The method as claimed in the preceding claim, **characterized in that** the molecularly imprinted polymer is used on an extraction column, for exemple an SPE cartridge.

8. The method as claimed in claim 6 or 7, in which said medium is a complex medium that may comprise, in addition to glutathione GSH and/or an analog thereof, at least one or more GSH adduct(s).

9. The method as claimed in claim 8, **characterized in that** it also comprises, as a preliminary to step (a), at least:
(i) a step of bringing said medium into contact with at least a first molecularly imprinted polymer capable of interacting with the GS adducts, and the glutathion and analogs thereof, under conditions suitable for extraction of the GSH adducts, of the glutathione and of analogs thereof,
(ii) separation of said molecularly imprinted polymer from the medium thus treated, and
(iii) formation of a medium enriched in said GSH adducts, in said glutathione and in said analogs thereof, by release of the GSH adducts, of the glutathione and of analogs thereof from the molecularly imprinted polymer resulting from step (ii).

10. The method as claimed in either one of claims 8 and 9, in which the complex medium is a medium for incubating an active molecule used in a glutathione-based toxicology test, for instance a medium for incubating a drug candidate.

11. The use of at least one molecularly imprinted polymer obtained according to claim 5 for the purposes of extraction, detection, separation, purification, absorption, adsorption, retention or controlled release of the free form of glutathione GSH and/or of an analog thereof.

12. The use of at least one molecularly imprinted polymer obtained according to claim 5 for the treatment of a medium that may comprise, or comprises, at least glutathione GSH and/or an analog thereof and GSH adducts.

## Patentansprüche

1. Verfahren zur Herstellung eines molekular geprägten Polymers (MIP), das zur selektiven Erkennung der reduzierten Form von Glutathion, GSH, oder eines Analogons vorgesehen ist, wobei das Verfahren mindestens umfasst:
- Copolymerisation eines Monomers oder von Monomeren, das/die zum Ausbilden einer Matrix der molekularen Prägung bestimmt ist/sind, mit mindestens einem Monomer der allgemeinen Formel (I):
X-(R)ₙ-Y-G (I),
worin.:
- X eine monovalente polymerisierbare Gruppe repräsentiert,
- n 0 oder 1 ist,
- R eine lineare, verzweigte oder cyclische, gesättigte oder ungesättigte, C₁-C₁₀-, insbesondere C₁-C₅-Kohlenwasserstoffkette repräsentiert, die gegebenenfalls durch ein oder mehrere Heteroatome, ausgewählt aus N, O und S, unterbrochen ist und gegebenenfalls substituiert ist,
- Y eine Funktion einer labilen Bindung repräsentiert, die die Sulfhydryl-Funktion des Glutathions GSH oder eines seiner Analoga mit einschließt,
- G den Rest des Glutathions GSH oder eines seiner entsprechenden Analoga repräsentiert,
unter Bedingungen, die für die Ausbildung der molekularen Prägung günstig sind, und
- Freisetzung des Restes G durch Spaltung der Bindungsfunktion Y.

2. Verfahren gemäß Anspruch 1, dadurch charakterisiert, dass X eine monovalente Gruppe, ausgewählt aus Vinyl-, Acryl-, Methacryl-, Alkyl-, Styrol-Gruppen oder einer jeglichen anderen ungesättigten Gruppe, die fähig ist, radikalisch zur reagieren, und den chemischen Gruppen, die eine Polykondensations- oder Sol-Gel-Reaktion erlauben, repräsentiert.

3. Verfahren gemäß einem jeglichen der vorstehenden Ansprüche, dadurch charakterisiert, dass Y eine Disulildfusktion -SS- repräsentiert.

4. Verfahren gemäß einem jeglichen der vorstehenden Ansprüche, dadurch charakterisiert, dass mindestens ein Monomer der allgemeinen Formel (I) N-Boc-Glutathiondisulfid-Methacrylat ist.

5. Molekular geprägtes Polymer (MIP), das durch das Verfahren gemäß einem jeglichen der vorstehenden Ansprüche erhältlich ist.

6. Verfahren zur Behandlung eines Mediums, das mindestens Glutathion GSH und/oder eines seiner Analoga umfassen kann oder umfasst, wobei das Verfahren mindestens umfasst:
(a) einen Schritt eines In-Kontakt-Bringens des Mediums mit mindestens einem gemäß Anspruch 5 erhaltenen molekular geprägten Polymer unter Bedingungen, die für eine Extraktion des Glutathions GSH und/oder seiner Analoga, falls vorhanden, durch die molekulare Prägung günstig sind,
(b) Abtrennung des molekular geprägten Polymers von dem so behandelten Medium und
(c) gegebenenfalls Freisetzung des Glutathions GSH und/oder seiner Analoga von dem sich aus Schritt (b) ergebenden molekular geprägten Polymer.

7. Verfahren gemäß dem vorstehenden Anspruch, dadurch charakterisiert, dass das molekular geprägte Polymer auf einer Extraktionssäule, z.B. einer SPE-Kartusche, verwendet wird.

8. Verfahren gemäß Anspruch 6 oder 7, wobei das Medium ein komplexes Medium ist, das neben dem Glutathion GSH und/oder einem seiner Analoga mindestens ein oder mehrere GSH-Addukte umfassen kann.

9. Verfahren gemäß Anspruch 8, dadurch charakterisiert, dass es ferner vor dem Schritt (a) mindestens umfasst:
(i) eine Schritt eines In-Kontakt-Bringens des Mediums mit mindestens einem ersten molekular geprägten Polymer, das geeignet ist, mit den GSH-Addukten, dem Glutathion und seinen Analoga zu interagieren, unter Bedingungen, die für eine Extraktion der GSH-Addukte, des Glutathions und seiner Analoga geeignet sind,
(ii) Abtrennung des molekular geprägten Polymers von dem so behandelten Medium und
(iii) Ausbildung eines Mediums, das an den GSH-Addukten, dem Glutathion und seinen Analoga angereicht ist, durch Freisetzung der GSH-Addukte, des Glutathions und seiner Analoga von dem sich aus Schritt (ii) ergebenden molekular geprägten Polymer.

10. Verfahren gemäß einem jeglichen der Ansprüche 8 oder 9, wobei das komplexe Medium ein Inkubationsmedium für ein aktives Molekül, das in einem Toxikologietest auf Glutathion verwendet wird, wie z.B. ein Inkubationsmedium für ein Kandidatenmedikament, ist.

11. Verwendung mindestens eines nach Anspruch 5 erhaltenen molekular geprägten Polymers zum Zweck einer Extraktion, Detektion, Abtrennung, Aufreinigung, Absorbtion, Adsorption, Retention oder kontrollierten Freisetzung der freien Form von Glutathion GSH und/oder eines seiner Analoga.

12. Verwendung mindestens eines nach Anspruch 5 erhaltenen molekular geprägten Polymers zur Behandlung eines Mediums, das mindestens Glutathion GSH und/oder eines seiner Analoga und GSH-Addukte umfassen kann oder umfasst.
